# EUROPÄISCHE PATENTANMELDUNG

(11) **EP 4 789 641 A2**
(43) Veröffentlichungstag der Anmeldung: **12.08.2026**
(21) Anmeldenummer: 26181606.0
(22) Anmeldetag: 28.04.2017
(51) Int. Cl.: A61F 2/78

(54) **PROTHESENSCHAFT**

(30) Priorität: 29.04.2016 DE 102016108043
(62) Dokumentnummer(n) der früheren Anmeldung(en) nach Art. 76 EPÜ: 17720476.5 / 3 448 324
(71) Anmelder: Otto Bock Healthcare Products GmbH, 1110 Wien (AT)
(72) Erfinder: Pawlik, Roland, 1110 Wien (AT); NIEUWENDIJK, Johan, 1110 Wien (AT)
(74) Vertreter: Gramm, Lins & Partner Patent- und Rechtsanwälte PartGmbB

(57) **Zusammenfassung**

Die Erfindung betrifft einen Prothesenschaft (1) mit einer Basis (10) für distale Anschlussmittel zur Anbindung einer Prothesenkomponente an den Prothesenschaft (1) und zumindest einer sich von der Basis (10) in Proximalrichtung erstreckenden Seitenwand (20), die einen in dem Prothesenschaft (1) aufzunehmenden Stumpf zumindest teilweise umschließt, wobei an der Basis (10) ein erster Träger (40) zur Festlegung einer ersten Seitenwand (21) und ein zweiter Träger (40) zur Festlegung einer zweiten Seitenwand (22) an der Basis (10) angeordnet ist, wobei die zumindest eine Seitenwand (21, 22) unterschiedliche Materialsteifigkeiten und unterschiedliche Materialien kombiniert aufweist.

## Beschreibung

Die Erfindung betrifft einen Prothesenschaft mit einer Basis für distale Anschlussmittel zur Anbringung einer Prothesenkomponente und zumindest einer sich von der Basis in Proximalrichtung erstreckenden Seitenwand, die einen in dem Prothesenschaft aufzunehmenden Stumpf zumindest teilweise umschließt. Ein solcher Prothesenschaft insbesondere zur Versorgung von Amputationsstümpfen oder von Dysmelien geeignet, um eine Anbindungsmöglichkeit für prothetische Komponenten wie Prothesenkniegelenke, Prothesenfüße oder Prothesenhände bereitzustellen.

Um Prothesenkomponenten möglichst sicher an einem Stumpf festzulegen, werden die meisten Prothesenschäfte individuell angefertigt. In der Regel wird ein Abdruck des Stumpfes genommen, von dem Abdruck wird ein Gipspositiv erstellt, das zur Formgebung des Prothesenschaftes verwendet wird, der in der Regel aus einem Kunststoff, gegebenenfalls einem faserverstärkten Kunststoff besteht. Auf das Positiv wird ein Platzhalter aufgebracht, um einen in der Regel zwischen dem Prothesenschaft und dem Stumpf anzuordnenden elastischen Liner nachzubilden, so dass zwischen dem Stumpfaußenumfang und dem Schaftinnenumfang ein Zwischenraum besteht. Der Prothesenschaft umschließt den Stumpf umfänglich und weist eine proximale Einstiegsöffnung und einen distalen Abschluss auf, an dem Anschlussmittel für weitere Protheseneinrichtungen angeordnet oder eingearbeitet sind. Diese Anschlussmittel sind beispielsweise Pyramidenadapter oder ähnliche Einrichtungen, mit denen beispielsweise Prothesenkniegelenke, Prothesenfüße oder Prothesenhände an dem Prothesenschaft festgelegt werden können. Durch diese Art und Weise der Fertigung erhält der Prothesenschaft den individuell passenden Umfang und die individuell passende Länge, eine Einstellbarkeit von Umfang oder Schaftlänge ist weder notwendig noch möglich.

Zur Anpassung an Stumpfvolumenschwankungen des Prothesenträgers können aufblasbare Luftkissen, Klettbänder oder eine Seilschnürung gemäß EP 2 629 705 A1 an einem Prothesenschaft oder einem Prothesenliner angeordnet sein. Die DE 10 2007 035 410 A1 betrifft einen Prothesenschaft zur Aufnahme eines Amputationsstumpfes einer Extremität mit Anschlussmitteln für eine distale Protheseneinrichtung mit zumindest einer Schale, die einen gekrümmten, offenen Querschnitt aufweist und deren Schalenenden im angelegten Zustand einander zumindest teilweise überlappen. Zumindest ein Spannmittel ist an der Schale angeordnet, das in Umfangsrichtung wirksam ist und die Schalenenden zueinander verspannt.

Aufgabe der vorliegenden Erfindung ist es, einen Prothesenschaft bereitzustellen, der mit industriell vorgefertigten Teilen herstellbar ist.

Erfindungsgemäß wird diese Aufgabe durch einen Prothesenschaft mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Der erfindungsgemäße Prothesenschaft mit einer Basis für distale Anschlussmittel zum Anschluss für eine Prothesenkomponente an den Prothesenschaft und zumindest einer sich von der Basis in Proximalrichtung erstreckenden Seitenwand, die einen in dem Prothesenschaft aufzunehmenden Stumpf zumindest teilweise umschließt, sieht vor, dass an der Basis zumindest ein Träger zur Festlegung der Seitenwand an der Basis angeordnet ist, wobei ein erster Träger zur Festlegung einer ersten Seitenwand und ein zweiter Träger zur Festlegung einer zweiten Seitenwand angeordnet ist, wobei zumindest eine Seitenwand unterschiedliche Materialsteifigkeiten und unterschiedliche Materialien kombiniert aufweist. Durch die erfindungsgemäße Aufteilung des Prothesenschaftes in einen distalen Prothesenschaftanteil in Gestalt einer Basis und einem proximalen Schaftanteil in Gestalt zumindest einer Seitenwand oder einer Seitenwandkomponente ist es möglich, die beiden Schaftanteile an-einander anzupassen und insbesondere die Schaftlänge einzustellen. Dies kann beispielsweise dadurch geschehen, dass der zumindest eine Träger und/oder die zumindest eine Seitenwand gekürzt wird und die gekürzte, an den jeweiligen Nutzer angepasste Kombination der Schaftanteile wieder miteinander verbunden werden. Dadurch ist es möglich, einen vorgefertigten Schaft an eine individuelle Stumpflänge anzupassen und die gewünschte Belastung an dem Stumpfende herzustellen, insbesondere bei einem Oberschenkelschaft, bei dem eine Abstützung sowohl an dem Sitzbein als auch an dem Stumpfende erfolgen kann. Durch die Längenanpassbarkeit kann die jeweils auf das Stumpfende aufgebrachte Druckkraft variiert werden, gegebenenfalls auch im Laufe der Tragedauer vergrößert werden oder aber an die jeweiligen Bedingungen an dem Stumpf über die Tragedauer angepasst werden. Dazu ist es vorteilhaft, wenn die Seitenwand oder Seitenwandkomponenten lösbar und reversibel an der Basis festlegbar sind. Anders als bei Maßanfertigungen ist bei der Verwendung von vorgefertigten Schaftanteilen, also von vorgefertigten Seitenwänden oder Seitenwandkomponenten und der Basis, eine Einstellbarkeit hinsichtlich der Länge notwendig.

Die Basis und die Seitenwand, die Seitenwände oder Seitenwandkomponenten sind in einer Weiterbildung der Erfindung so ausgebildet, dass sie zueinander in unterschiedlichen Positionen festlegbar oder fixierbar sind. Dadurch ist es beispielsweise möglich, dass eine Seitenwand von dem distalen Ende des Trägers aus gesehen höher als die andere Seitenwand an dem Träger befestigt wird, so dass sich eine individuelle Anpassung an den Stumpf durch einfaches Verschieben und Fixieren der Seitenwand, der Seitenwände oder der Seitenwandkomponenten an dem Träger erreichen lässt.

Eine Weiterbildung der Erfindung sieht vor, dass die Basis als eine formstabile Kappe oder formstabile Platte an seinem distalen Ende ausgebildet ist oder eine solche Kappe oder Platte aufweist, so dass tragende, distale Schaftanteile an der Basis ausgebildet sind oder die Basis solche tragenden Schaftanteile aufweist. An dieser formstabilen Kappe oder Platte kann sich einerseits der Stumpf abstützen und andererseits können dort die Anschlussmittel für die distalen Prothesenkomponenten angeordnet, ausgebildet oder fixiert werden.

Die Basis kann Halteeinrichtungen für einen Liner, der an dem Stumpf angelegt ist, um ein Interface zwischen dem Stumpf und dem in der Regel formstabilen Prothesenschaft auszubilden, aufweisen. Grundsätzlich ist es auch möglich, dass Halteeinrichtungen für den Stumpf an dem Prothesenschaft ausgebildet oder angeordnet sind. Die Halteeinrichtungen für den Liner können als Aufnahmeelemente für einen Zapfen ausgebildet sein, also einen sogenannten Pinlock ausbilden. Ebenfalls ist es möglich, durch andere Formschlusselemente oder Kraftschlusselemente den Liner an der Basis zu sichern. Beispielsweise können an der Basis Magnete angeordnet sein, um einen entsprechend konfigurierten Liner, der ebenfalls Magnete oder ferromagnetische Elemente aufweist, an der Basis zu fixieren. Ebenso ist es möglich, dass an der Seitenwand oder den Seitenwandkomponenten Klemmelemente oder Klemmeinrichtungen, Halteelemente oder Fixiereinrichtungen angeordnet oder ausgebildet sind, über die der Liner entlang seiner Längserstreckung an der Seitenwand festgelegt, geklemmt, fixiert oder gesichert wird. Auch diese Halteelemente an der Innenseite der Seitenwand können als Formschlusselemente oder Kraftschlusselemente, insbesondere als Vorsprünge, Klettverschlüsse, ein Steigfell, Strichvelours oder als Magnete ausgebildet sein. Es können auch Kombinationen aus Formschlusselementen und Kraftschlusselementen sowohl an der Basis als auch an der Seitenwand oder den Seitenwandkomponenten angeordnet oder ausgebildet sein.

Eine Weiterbildung der Erfindung sieht vor, dass die Seitenwand formschlüssig an der Basis festgelegt ist. Die Seitenwand oder die Seitenwandkomponenten können an der Basis festgeschraubt, über Rastelemente fixiert, über einen Bajonettverschluss oder Formschlusselemente in Gestalt von Klipsen oder anderen Rasteinrichtungen fixiert werden. Ebenfalls kann die Seitenwand mit der Basis vernietet sein. Alternativ oder ergänzend kann die Seitenwand klemmend an der Basis oder dem Träger festgelegt sein, um eine stufenlose Einstellbarkeit der Position der Seitenwand relativ zu der Basis zu realisieren.

Die Seitenwand kann aus mehreren Seitenwandkomponenten ausgebildet sein und eine Spanneinrichtung aufweisen, über die die Seitenwandkomponenten zueinander ausgerichtet oder gegeneinander verspannt werden können. Alternativ oder ergänzend können zwei oder mehr Seitenwände ausgebildet sein, die über zumindest eine Spanneinrichtung zueinander ausgerichtet oder zueinander verspannt werden können. Die Spanneinrichtung kann als Gurt ausgebildet sein, der an beiden Seitenwandkomponenten festgelegt ist und über eine Längenveränderung eine Weiteneinstellbarkeit der beiden oder mehreren Seitenwandkomponenten zueinander ermöglicht. Grundsätzlich ist es auch möglich, dass die Seitenwandkomponenten zueinander verschiebbar an der Basis gelagert sind, um eine Anpassung an unterschiedliche Stumpfgrößen zu ermöglichen. Die Träger können einander gegenüberliegend angeordnet sein, beispielsweise auf der medialen und der lateralen Seite, wobei es grundsätzlich möglich und vorgesehen ist, dass die Träger an anderen Positionen angeordnet sind, z.B. anterior und posterior. In einer Ausgestaltung der Erfindung ist vorgesehen, dass zumindest ein Träger an der medialen Seite angeordnet ist.

Eine Weiterbildung der Erfindung sieht vor, dass zumindest ein Träger klappbar an der Basis angeordnet ist. Werden mehrere Träger an der Basis angeordnet oder ausgebildet, können entweder alle Träger klappbar an der Basis angeordnet sein oder aber zumindest eines starr mit der Basis verbunden sein, so dass dieser Träger als Referenzelement dient. Zum Einstellen der Prothesenschaftweite und zum erleichterten Anlegen des Prothesenschaftes kann ein Träger mit einer Seitenwandkomponente abgeklappt und dadurch eine Umfangserweiterung bewirkt werden. Der Stumpf mit dem Liner wird dann in den Prothesenschaft eingeführt, beispielsweise mit dem distalen Ende an die Innenseite der distalen Basis aufgesetzt. Die fest mit der Basis gekoppelte Seitenwandkomponente wird an den Liner angelegt und die klappbar an der Basis gelagerte Seitenwandkomponente wird in Richtung auf den Liner herangeklappt, um so eine Fixierung des Stumpfes mit dem Liner in dem Prothesenschaft zu ermöglichen. Die beiden Seitenwandkomponenten können einander in den Randbereichen überdecken, so dass der Prothesenschaft den aufzunehmenden Stumpf vollständig umschließt, grundsätzlich ist es auch möglich, dass ein Abstand zwischen den Seitenrändern der Seitenwandkomponenten vorhanden ist, sofern die Formgebung und Formstabilität der Seitenwandkomponenten ausreicht, eine sichere Aufnahme des Stumpfes in dem Prothesenschaft zu gewährleisten.

Der Träger oder die Träger können als Schiene ausgebildet sein, die sich in proximaler Richtung von der Basis weg erstrecken. Bevorzugt sind die Schienen kürzer als die Seitenwand oder die Seitenwandkomponenten ausgebildet und können gekürzt werden, so dass eine geringere Gesamtschaftlänge erzielt werden kann.

In dem Träger, insbesondere in der Schiene, können Einrichtungen zum formschlüssigen Fixieren der Seitenwand angeordnet oder ausgebildet sein, insbesondere sind diese Einrichtungen in diskreten Abständen ausgebildet, um eine Zuordnung in diskreten Abständen zueinander zu erleichtern. Alternativ hierzu ist es möglich, über eine Langlochausgestaltung entweder an dem Träger oder an der Seitenwand eine verschiebliche Lagerung mit einer klemmenden Halterung vorzusehen. Neben einer relativ groben Rasterung über Bohrungen, die zueinander ausgerichtet sind und in entsprechend großen Abständen eine Längenkürzen ermöglichen, kann an dem Träger eine Feinrasterung ausgebildet sein, die mit korrespondierenden Vorsprüngen an der Seitenwand oder den Seitenwandkomponenten in Eingriff tritt, so dass eine Feineinstellung der Länge möglich ist.

Die Seitenwand oder die Seitenwandkomponente können Führungen oder Aufnahmen aufweisen, in die der Träger einführbar ist. Bei einer Ausgestaltung des Trägers als Schiene sind die Führungen als Schienenaufnahmen ausgebildet, die eine Längsverschieblichkeit und ein Einlegen von außen ermöglichen, so dass insgesamt drei longitudinale Freiheitsgrade vorhanden sind. Durch eine entsprechende Ausgestaltung der Schienenaufnahme oder Aufnahme des Trägers kann eine Verschieblichkeit nur noch in Längserstreckung des Trägers bzw. der Schienenaufnahme erfolgen, beispielsweise durch eine trapezartige Ausgestaltung der Schiene oder aber durch eine geschlossene Führung in Gestalt einer Buchse. Die Führungen und die Träger sind bevorzugt gleich lang, so dass bei einem vollständigen Einschieben des Trägers in der Führung die Führung vollständig ausgefüllt ist. Um die Schaftlänge zu kürzen, müssen dann beide Komponenten gleichmäßig gekürzt werden.

An der Seitenwand und/oder dem Träger können Markierungen oder Materialschwächungen angeordnet oder ausgebildet sein, über die Sollbruchlinien, Sollschnittlinien oder Trennlinienvorgaben an dem Träger und/oder der Seitenwand oder Seitenwandkomponente ausgebildet werden. Insbesondere bei den Trägern ist eine solche Markierung oder Materialschwächung im proximalen Bereich angeordnet, bei der Seitenwand oder den Seitenwandkomponenten sind diese insbesondere im distalen Bereich angeordnet, um eine Kürzbarkeit und Längeneinstellbarkeit zu erleichtern. Die Markierungen oder Materialschwächungen sind bevorzugt umlaufend oder zumindest quer zur Längserstreckung mit der jeweiligen Komponente ausgebildet.

Die Seitenwand oder die Seitenwände können mit unterschiedlichen Materialsteifigkeit und/oder unterschiedlichen Materialstärken ausgebildet sein. Die zumindest eine Seitenwand muss also nicht aus einem einzigen, homogenen Material in einer gleichbleibenden Materialstärke bestehen, vielmehr ist es in einer Variante der Erfindung vorgesehen, dass unterschiedliche Materialien kombiniert werden, dass unterschiedliche Materialstärken in einer Seitenwand oder Seitenwandkomponente vorhanden sind und/oder dass Materialen bereichsweise unterschiedlich behandelt werden, um unterschiedlichen Eigenschaften, insbesondere Steifigkeiten zu erzielen. So können im Bereich des Trägers die Seitenwände starr ausgeführt sein, während die übrigen Bereiche elastisch oder bereichsweise mit flexiblen Stellen ausgerüstet sein können.

An der Basis können zumindest zwei Träger angeordnet und ausgebildet sein, von denen eine medial und eine lateral an der Basis orientiert ist, so dass eine gute seitliche Führung vorhanden ist. Über die medial-lateral-Anordnung des Trägers wird der Tragekomfort des Prothesenschaftes nicht oder nur unwesentlich beeinträchtigt, da die formstabilen Träger, die beispielsweise aus einem Stahl oder Aluminium ausgebildet sein können, sich beim Sitzen oder beim Stehen mit einer Belastung des proximalen Prothesenschaftrandes an dem Sitzbein nicht bemerkbar machen.

Die Seitenwand kann einen offenen Querschnitt aufweisen, so dass eine Umfangseinstellung über einen in Umfangsrichtung wirkendes Stellsystem einfach realisieren lässt. Neben einer Längeneinstellbarkeit kann durch ein Spannsystem, mit dem mehrere Seitenwandkomponenten gegeneinander verspannt werden oder aber auch eine Seitenwand mit einem offenen Querschnitt gegeneinander eingestellt wird, so dass sich ein veränderbarer Überlappungsbereich der in Umfangsrichtung orientierten Ränder ergibt, realisiert werden kann.

Nachfolgend werden Ausführungsbeispiele der Erfindung anhand der beigefügten Figuren näher erläutert. Es zeigen:
- Figur 1-: die Explosionsdarstellung
- Figur 2-: eine schematische Seitenansicht im nicht montierten Zustand; sowie
- Figur 3-: einen Prothesenschaft im endmontierten Zustand.

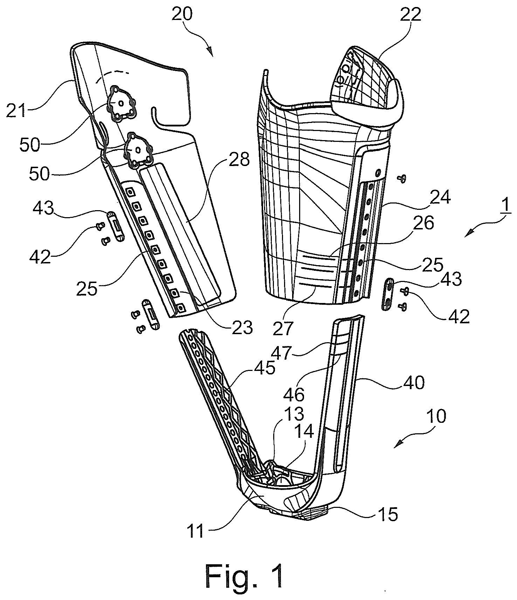

Figur 1 zeigt in einer Explosionsdarstellung einen Prothesenschaft 1 mit einer Basis 10, an dem zwei Seitenwandkomponenten 21, 22 lösbar befestigt sind. Im montierten und angelegten Zustand umgeben die beiden Seitenwandkomponenten 21, 22 den nicht dargestellten Stumpf vollumfänglich und bilden eine den Stumpf umschließende Seitenwand 20. In einer alternativen Ausführungsform kann die Seitenwand 20 auch einteilig ausgebildet sein und entweder einen geschlossenen Querschnitt oder einen offenen Querschnitt aufweisen.

Die Basis 10 in dem dargestellten Ausführungsbeispiel weist an seinem distalen Ende eine formstabile Kappe 11 auf, dessen distaler Seite nicht dargestellte Anschlussmittel für eine weitere Prothesenkomponente, beispielsweise ein Prothesenkniegelenk festlegbar sind. Diese formstabile Kappe 11 dient als Auflage für das nicht dargestellte Stumpfende und weist zumindest eine Halteeinrichtung 14 für eine Festlegung oder Sicherung des Stumpfes oder an einem Stumpf angelegten Liners an dem Prothesenschaft 1 auf. Diese Halteeinrichtung 14 ist im dargestellten Ausführungsbeispiel als Klettverschlusskomponente ausgebildet, in einer alternativen Ausführungsform können andere Formschlusseinrichtungen oder auch eine Magnetsicherung eingebaut sein, um über korrespondierende Formschlusselemente oder ferromagnetische Komponenten eine Sicherung des distalen Endes des Liners an der Basis 10 und damit an dem Prothesenschaft zu bewirken.

Von der formstabilen Kappe 10 erstrecken sich in Proximalrichtung zwei Träger 40, die als Schienen ausgebildet sind. Die Träger 40 sind im angelegten Zustand des Prothesenschaftes 1 auf der medialen und lateralen Seite des Stumpfes positioniert und können entweder eine gleiche Länge oder aber unterschiedliche Längen aufweisen, wobei auf der lateralen Seite bevorzugt eine größere Länge vorgesehen ist. Innerhalb der als Schienen ausgebildeten Träger 40 sind Durchgangsbohrungen 45 als Formschlusselemente zur Festlegung der Seitenwandkomponenten 21, 22 angeordnet. Die Durchgangsbohrungen 45 können mit einem Gewinde versehen sein und sind in einem definierten Abstand zueinander hintereinander in Längserstreckung der Träger 40 angeordnet. Der lateral angeordnete Träger 40 ist über ein Scharnier 13 klappbar an der Basis 10 angeordnet, der mediale Träger 40 ist einstückig an der distalen Kappe 41 ausgebildet, so dass sich ein L-förmiger, starrer Grundkörper ausbildet. Neben einer schwenkbaren Lagerung des medialen Trägers 40 kann dieses auch verschieblich an der Basis 10 angeordnet sein, um eine Anpassbarkeit der Stumpfweite im distalen Bereich zu ermöglichen. Grundsätzlich ist es auch möglich, beide Träger 40 klappbar auszugestalten oder aber auch den medialen Träger 40 starr an der formstabilen Kappe 11 anzuordnen oder damit auszubilden. Auch sind mehr als zwei Träger 40 an der Basis ausbildbar oder anordbar.

Die Seitenwandkomponenten 21, 22 und damit die gesamte Seitenwand 20 sind lösbar an der Basis 10 befestigt. Dazu sind an den Außenseiten der Seitenwandkomponenten 21, 22 Führungen 23, 24 für die Träger 40 befestigt. Die Führungen 23, 24 sind als Schienenaufnahmen ausgebildet, die korrespondierend zu den schienenartigen Ausgestaltungen der Träger 40 ausgebildet sind und diese in sich aufnehmen können. Die Führungen 23, 24 können C-förmig ausgebildet sein, wobei der Querschnitt der Führungen 23, 24 entweder ein seitliches Einführen der Träger 40 zulässt oder aber aufeinander zu gerichtete Dachschenkel aufweist, so dass die jeweiligen Träger 40 nur in Längserstreckung in die entsprechende Führung 23, 24 eingeschoben werden kann. Die Seitenwände der Führung 23, 24 kann auch zueinander geneigt ausgebildet sein, so dass die Träger 40 sowohl in Medialrichtung als auch in Lateralrichtung fixiert jedoch weiterhin in Längserstreckung der Träger 40 verschieblich in den Führungen 23, 24 geführt sind.

In den Führungen 23, 24 sind korrespondierend zu den Durchgangsbohrungen 45 in den Trägern 40 Bohrungen mit Innengewinden 25 angeordnet, die in Abständen zueinander in Längserstreckung hintereinander angeordnet sind, in denen sie mit den Durchgangsbohrungen 45 fluchten. Über Formschlusselemente 42 in Gestalt von Schrauben werden die Seitenwandkomponenten 21, 22 mit dem jeweiligen Träger 40 verbunden. Dazu werden die Träger 40 in die Führungen 23, 24 eingeführt, bis die gewünschte Länge erreicht ist. Die Durchgangsbohrungen 45 und die Innengewinde 25 innerhalb der Führungen 23, 24 werden zueinander ausgerichtet und die Schrauben 42 hindurchgesteckt und fixiert. Dadurch wird eine formschlüssige, lösbare Festlegung der Seitenwand 20 an der Basis 10 erreicht. Die Durchgangsbohrungen 45 können auch als Langlöcher ausgebildet sein, um eine quasi - stufenlose Einstellung der Position der Seitenwandkomponenten 21, 22 relativ zu der Basis 10 zu ermöglichen. Es kann auch ein sich über die Gesamtlänge erstreckendes Langloch ausgebildet sein, so dass neben einer formschlüssigen Fixierung der Seitenwand 20 an den Trägern 40 die Längsverschieblichkeit entlang der Führungen 23, 24 klemmend über die Schrauben 42 und eine Unterlegscheibe 43 blockiert wird.

Im distalen Bereich der Seitenwandkomponenten 21, 22 sind Markierungen 26 und Materialschwächungen 27 quer zur Längserstreckung ausgerichtet angebracht, um bei Notwendigkeit die Gesamtlänge kürzen zu können. Die Materialschwächungen 27 können sich über den gesamten Umfang der Seitenwandkomponente 21, 22 erstrecken, bei einer einteiligen Ausgestaltung der Seitenwand 20 vollumfänglich. Die Markierungen 26 sind bevorzugt in gleichmäßigen Abständen zueinander orientiert und können auch mit einer Skala versehen sein, um eine Längenanpassung zu erleichtern. Korrespondierende Markierungen 46 und Materialschwächungen 47 sind an den Trägern 40, vorzugsweise an deren proximalen Enden angeordnet und ermöglichen eine individuelle Anpassbarkeit der Länge des gesamten Prothesenschaftes 1.

Die Seitenwandkomponenten 20, 21 bilden als Seitenwand 20 einen proximalen Schaftanteil, die Basis 10 mit den Trägern 40 bildet einen distalen Schaftanteil, die längsverschieblich zueinander und aneinander festlegbar ausgebildet und angeordnet sind, so dass sie entweder stufenlos oder stufenverstellbar und fixierbar zueinander sind. Durch ein Kürzen des distalen Schaftanteils in Gestalt der Basis 10 und/oder des proximalen Schaftanteils in Gestalt der Seitenwand 20 kann eine Verringerung der Schaftlänge erzielt werden, wodurch es möglich ist, industriell vorgefertigte Schaftteile einzusetzen, um einen vorgefertigten Schaft an individuelle Stumpflängen anpassen zu können. Auch kann die gewünschte Belastung auf das Stumpfende angepasst und eingestellt werden.

An der lateralen Seitenwandkomponente 21 ist eine Spanneinrichtung 50 angeordnet, über die beispielsweise über ein Schnur-Kabel-Gurtsystem die Schaftweite variiert werden kann. Aufgrund der zweiteiligen Ausgestaltung der Seitenwand 20 im dargestellten Ausführungsbeispiel sowohl der klappbaren Lagerung der lateralen Seitenwandkomponente 21 an der Basis 10 ist es möglich und notwendig, die Position der Seitenwandkomponenten 21, 22 zueinander in Umfangsrichtung zu fixieren. Über die Klappbarkeit der Basis 10 und die dadurch erreichbare Aufweitbarkeit des Prothesenschaftes 1 ist es möglich, dass Einsteigen in den Prothesenschaft zu erleichtern. Ebenfalls ist es dadurch möglich, Formschlusselemente 28 an der Innenseite der Seitenwand anzuordnen oder auszubilden, die eine Bewegung in Proximalrichtung behindern oder verhindern. So kann an der Innenseite der Seitenwand 20 ein Klettverschluss, ein Steigfell oder eine Oberflächenstrukturierung angeordnet oder ausgebildet sein, die formschlüssig in eine entsprechende Struktur an dem Liner eingreift. Durch die Aufklappbewegung kann ein Abschälvorgang oder außer Eingriff Bringen erfolgen, so dass eine entsprechende Trennung von Prothesenschaft 1 und Liner leicht möglich ist.

In dem dargestellten Ausführungsbeispiel der Figur 1 sind die Seitenwandkomponenten 21, 22 unterschiedlich ausgestaltet. Die mediale Seitenwandkomponente 22 erstreckt sich über mehr als die Hälfte des Gesamtumfanges, hier 3/4 des Gesamtumfanges, während die laterale Seitenwandkomponente 21 so bemessen ist, dass eine Umfangsergänzung vorliegt, so dass der Prothesenstumpf vollumfänglich umschlossen werden kann. Die Seitenkanten der Seitenwandkomponenten 21, 22 können im angelegten und zueinander festgelegten Zustand einander überlappen und ermöglichen dadurch eine Weiteneinstellbarkeit auch im Verlauf des Tragens, wenn Stumpfvolumenschwankungen vorliegen.

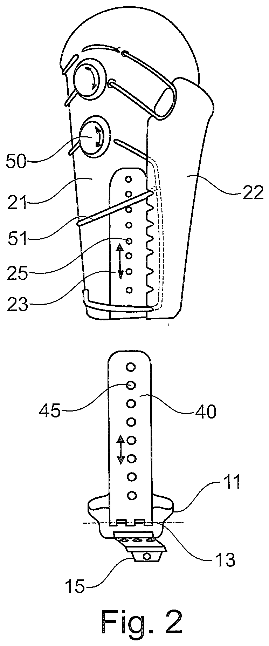

Ein Prothesenschaft 1 mit Basis 10 und Seitenwand 20 vor der Montage ist in der Figur 2 dargestellt, in der an die Basis 10 an dessen distalen Ende die distalen Anschlussmittel 15 für eine Prothesenkomponenten beispielsweise in Gestalt eines Prothesenkniegelenkes angeordnet sind. Das distale Anschlussmittel 15 ist als Aufnahme eines Pyramidenadapters ausgebildet.

Die Seitenwand 20 ist über die Spanneinrichtung 50 umfänglich geschlossen ausgebildet. Die Spanneinrichtung 50 ist als sogenanntes Disk-System ausgebildet, bei dem durch Drehen eines Rades ein Gurtsystem oder Kabelsystem 51 gespannt und entspannt werden kann. Durch das Spannen des Gurtsystems oder Kabelsystems 51 kann eine Weitenregulierung erfolgen.

Zur Montage der Seitenwand 20 an der Basis werden die Träger 40 in die Führungen eingeschoben, wobei im dargestellten Ausführungsbeispiel aufgrund der Seitenansicht aus Lateralrichtung nur eine Führung 24 und ein als Schiene ausgebildeter Träger 40 sichtbar ist. Nach Erreichen der gewünschten Position werden die Schrauben 42 durch die Durchgangsbohrungen 45 und in die Gewinde 25 in den Führungen 23, 24 eingeschraubt.

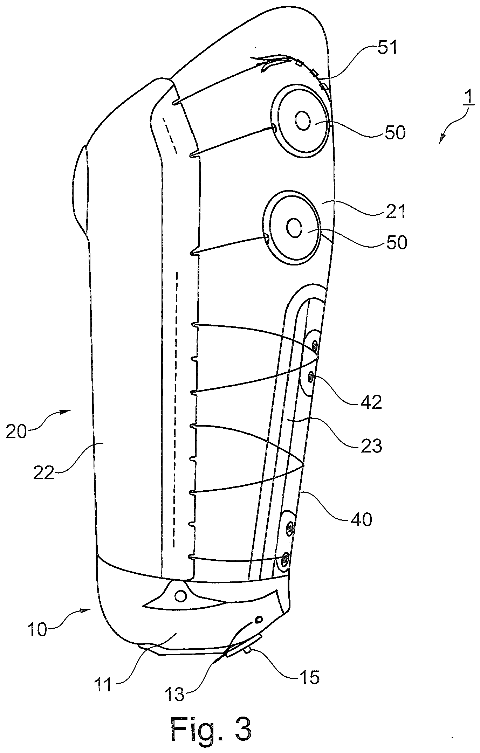

Ein fertig montierter Prothesenschaft 1 ist in der Figur 3 dargestellt. Es ist die distale, formstabile Kappe 11, die Scharniereinrichtung 13 für den lateralen Träger 40 sowie die Seitenwand 20 aus einer lateralen Seitenwandkomponente 21 und einer medialen Seitenwandkomponenten 22 zu erkennen. Die Spanneinrichtung 50 weist im dargestellten Ausführungsbeispiel zwei Drehmechanismen auf, über die zwei Kabel 51 in ihrer effektiven Länge veränderbar sind. Die Kabel oder Bänder 51 sind an dem einander gegenüberliegenden Seitenrändern der medialen Seitenwandkomponente 22 angeordnet und bewirken durch eine Verkürzung eine Umfangsverringerung. Durch entgegengesetztes Drehen der Spanneinrichtungen 50 kann der Prothesenschaftumfang erweitert werden. Die laterale Seitenwandkomponente 21 ist an der Basis 10 über insgesamt vier Schrauben 42 lösbar fixiert. Die Schrauben 42 sind durch die Durchgangsbohrungen 45 in dem Träger 40 hindurch geführt und in die Innengewinde 25 innerhalb der Führung 23 eingeschraubt. Durch die Unterlegscheibe 43 ergibt sich eine zusätzliche Klemmwirkung.

## Patentansprüche

1. Prothesenschaft (1) mit einer Basis (10) für distale Anschlussmittel zur Anbindung einer Prothesenkomponente an den Prothesenschaft (1) und zumindest einer sich von der Basis (10) in Proximalrichtung erstreckenden Seitenwand (20), die einen in dem Prothesenschaft (1) aufzunehmenden Stumpf zumindest teilweise umschließt, **dadurch gekennzeichnet, dass** an der Basis (10) ein erster Träger (40) zur Festlegung einer ersten Seitenwand (21) und ein zweiter Träger (40) zur Festlegung einer zweiten Seitenwand (22) an der Basis (10) angeordnet ist, wobei die zumindest eine Seitenwand (21, 22) unterschiedliche Materialsteifigkeiten und unterschiedliche Materialien kombiniert aufweist.

2. Prothesenschaft nach Anspruch 1, **dadurch gekennzeichnet, dass** die zumindest eine Seitenwand (20) oder die Seitenwände (20) an der Basis (10) an unterschiedlichen Positionen festlegbar sind.

3. Prothesenschaft nach Anspruch 1 oder 2, **dadurch gekennzeichnet, dass** die Basis (10) eine formstabile Kappe (11) oder Platte an seinem distalen Ende aufweist.

4. Prothesenschaft nach einem der voranstehenden Ansprüchen, **dadurch gekennzeichnet, dass** an der Basis (10) zumindest eine Halteeinrichtung (14) für einen Liner oder einen Stumpf angeordnet ist.

5. Prothesenschaft nach Anspruch 4, **dadurch gekennzeichnet, dass** die Halteeinrichtung (14) als Magnet ausgebildet ist.

6. Prothesenschaft nach einem der voranstehenden Ansprüche, **dadurch gekennzeichnet, dass** die Seitenwände (20, 22) im Bereich der Träger (40) starr ausgeführt sind, während die übrigen Bereiche elastisch oder bereichsweise mit flexiblen Stellen ausgerüstet sind.

7. Prothesenschaft nach einem der voranstehenden Ansprüche, **dadurch gekennzeichnet, dass** die Seitenwand (20) aus mehreren Komponenten (21, 22) ausgebildet ist oder zwei oder mehre Seitenwände (20) ausgebildet sind, die über zumindest eine Spanneinrichtung (50) zueinander ausgerichtet oder zueinander verspannt werden können.

8. Prothesenschaft nach einem der voranstehenden Ansprüche, **dadurch gekennzeichnet, dass** zumindest ein Träger (40) klappbar an der Basis (10) angeordnet ist.

9. Prothesenschaft nach einem der voranstehenden Ansprüche, **dadurch gekennzeichnet, dass** der Träger (40) als eine sich in Proximalrichtung erstreckende Schiene ausgebildet ist.

10. Prothesenschaft nach einem der voranstehenden Ansprüche, **dadurch gekennzeichnet, dass** an dem Träger (40) Formschlusselemente (45) zur Festlegung der Seitenwand (20) angeordnet sind.

11. Prothesenschaft nach einem der voranstehenden Ansprüche, **dadurch gekennzeichnet, dass** an der Seitenwand (20) zumindest eine Führung (23, 24) zur Aufnahme des Trägers (40) angeordnet ist.

12. Prothesenschaft nach einem der voranstehenden Ansprüche, **dadurch gekennzeichnet, dass** die Seitenwand (20) über Formschlusselemente (42) an dem Träger (40) festgelegt ist.

13. Prothesenschaft nach einem der voranstehenden Ansprüche, **dadurch gekennzeichnet, dass** an der Seitenwand (20) und/oder dem Träger (40) Markierungen (26, 46) und/oder Materialschwächungen (27, 47) als Sollbruchlinien oder Sollschnittlinien angeordnet oder ausgebildet sind.

14. Prothesenschaft nach einem der voranstehenden Ansprüche, **dadurch gekennzeichnet, dass** zumindest ein Träger (40) medial oder ein Träger (40) medial und ein Träger (40) lateral an der Basis (10) angeordnet ist.

15. Prothesenschaft nach einem der voranstehenden Ansprüche, **dadurch gekennzeichnet, dass** die Seitenwand (20) als in Umfangsrichtung offener Querschnitt mit einander überlappenden Randbereichen ausgebildet ist.

16. Prothesenschaft nach einem der voranstehenden Ansprüche, **dadurch gekennzeichnet, dass** die zumindest eine Seitenwand (20) unterschiedliche Materialstärken aufweist.
